(19) Europäisches Patentamt
European Patent Office
Office européen des brevets

(11) EP 1 449 840 A1

(12) EUROPEAN PATENT APPLICATION

(43) Date of publication:
**25.08.2004 Bulletin 2004/35**

(51) Int Cl.$^7$: **C07D 315/00**

(21) Application number: **04250834.1**

(22) Date of filing: **17.02.2004**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **19.02.2003 JP 2003041416**
**02.09.2003 JP 2003310279**

(71) Applicant: **Kyushu University**
**Fukuoka City, Fukuoka Pref. (JP)**

(72) Inventors:
- **Katsuki, Tsutomu, c/o Kyushu University**
  **Fukuoka City, Fukuoka Pref. (JP)**
- **Ito, Katsuji, c/o Fukuoka University of Education**
  **Munakata City, Fukuoka Pref. (JP)**
- **Ishii, Ayako**
  **Munakata City, Fukuoka Pref. (JP)**
- **Kuroda, Tomomi**
  **Munakata City, Fukuoka Pref. (JP)**

(74) Representative: **Whalley, Kevin**
**MARKS & CLERK,**
**57-60 Lincoln's Inn Fields**
**London WC2A 3LS (GB)**

# (54) Method of producing optically active lactone compound

(57) An optically active lactone compound is produced by using a complex in which Pd or Pt is a central metal and a compound having a specified structure is a ligand as a catalyst, and subjecting a cyclic ketone compound to a Baeyer-Villiger oxidation with a specified oxidizer.

EP 1 449 840 A1

## Description

### BACKGROUND OF THE INVENTION

### 1. Field of the Invention

This invention relates to a method of producing an optically active lactone compound. More particularly, the invention relates to a method of producing an optically active lactone compound, which comprises using a Pd or Pt complex having a specified ligand as a catalyst, and conducting a Baeyer-Villiger oxidation of a cyclic ketone compound with an oxidizer. Such an optically active lactone compound is useful for the synthesis of medicines and agrochemicals.

### 2. Description of the Related Art

The Baeyer-Villiger oxidation is a potent tool for transforming carbonyl compounds to lactones or esters and is widely used in organic synthesis. Naturally, its asymmetric version, i.e. asymmetric Baeyer-Villiger oxidation is expected to be a useful method for the synthesis of optically active lactones that are efficient as a chiral building block. Therefore, much effort has been recently directed toward study of the asymmetric Baeyer-Villiger oxidation.

In 1994, Bolm et al. and Strukul et al. independently reported a matal-mediated asymmetric Baeyer-Villiger oxidation (Bolm C., Schlingloff G. and Weickhardt K., Angew. Chem. Int. Ed. Engl., 1994, 33, 1848-1849; Gusso A., Baccin C., Pinna F. and Strukul G., Organometallics, 1994, 13, 3442-3451). Since then, several catalytic asymmetric Baeyer-Villiger oxidations were reported, but satisfactory enantioselectivity has not been achieved except for a few examples (Bolm C., Luong K. K. and Schlingloff G., Synlett, 1997, 1151-1152; Paneghetti C., Gavagnin R., Pinna F. and Strukul G., Organometallics, 1999, 18, 5057; Bolm C., Beckmann O., Cosp A. and Palazzi C., Synlett, 2001, 1461-1463; Bolm C., Beckmann O. and Plazzi C., Can. J. Chem., 2001, 79, 1593-1597).

### SUMMARY OF THE INVENTION

It is, therefore, an object of the invention to provide a method of producing a lactone compound with a high optical purity by conducting the asymmetric Baeyer-Villiger oxidation of a prochiral cyclic ketone compound in a highly enantioselective manner.

The inventors have made various studies in order to achieve the above object and discovered that a lactone compound with a high optical purity can be produced by using a complex, in which Pd or Pt is a central metal and a compound having a specified structure is a ligand, as a catalyst and subjecting a cyclic ketone compound to a Baeyer-Villiger oxidation with a specified oxidizer, and as a result, the invention has been accomplished.

According to the invention, there is the provision of a method of producing an optically active lactone compound, which comprises using as a catalyst a complex in which Pd or Pt is a central metal and a ligand is selected from the group consisting of a compound represented by the following formula (I), (II), (III) or (IV) and its enantiomer, and subjecting a cyclic ketone compound to a Baeyer-Villiger oxidation with at least one oxidizer selected from the group consisting of hydrogen peroxide, urea-hydrogen peroxide adduct (UHP) and alkyl hydroperoxide:

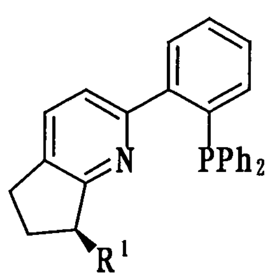

· · · · · (I)

(wherein $R^1$ is a linear or branched alkyl group having a carbon number of 1 to 10 provided that a hydrogen atom of the alkyl group may be substituted with t-butyldimethylsiloxy (OTBS) group);

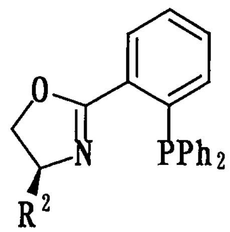

· · · · · (II)

(wherein $R^2$ is an aryl group having a carbon number of 6 to 10 or a linear or branched alkyl group having a carbon number of 1 to 10);

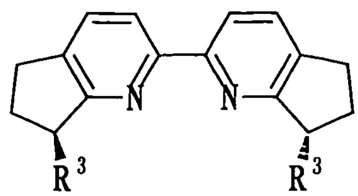

· · · · · (III)

(wherein $R^3$ is independently a linear or branched alkyl group having a carbon number of 1 to 10 provided that a hydrogen atom of the alkyl group may be substituted with t-butyldimethylsiloxy (OTBS) group);

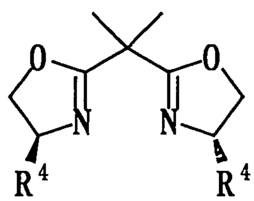

· · · · · (IV)

(wherein $R^4$ is independently an aralkyl group having a carbon number of 7 to 11 or a linear or branched alkyl group having a carbon number of 1 to 10).

In a preferable embodiment of the invention, a counter ion of the complex is $SbF_6^-$ or $BF_4^-$.

In another preferable embodiment of the invention, the central metal of the complex is Pd.

In the other preferable embodiment of the invention, the ligand of the complex is a compound represented by the formula (I) in which $R^1$ is i-propyl group or 1-methyl-1-(t-butyldimethylsiloxy) ethyl group, or its enantiomer. As $R^1$ in the formula (I), i-propyl group is particularly preferable.

In a further preferable embodiment of the invention, the ligand of the complex is a compound represented by the formula (II) in which $R^2$ is phenyl group or t-butyl group, or its enantiomer.

In a still further preferable embodiment of the invention, the ligand of the complex is a compound represented by the formula (III) in which $R^3$ is t-butyldimethylsiloxymethyl group or 1-methyl-1-(t-butyldimethylsiloxy)ethyl group, or its enantiomer.

In a further preferable embodiment of the invention, the ligand of the complex is a compound represented by the formula (IV) in which $R^4$ is benzyl group or t-butyl group, or its enantiomer.

In a still further preferable embodiment of the invention, the cyclic ketone compound is represented by the following formula (V), (VI) or (VII):

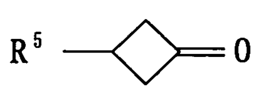

· · · · · (V)

(wherein $R^5$ is a substituted or non-substituted alkyl group having a carbon number of 1 to 20 or a substituted or non-substituted aryl group having a carbon number of 6 to 15);

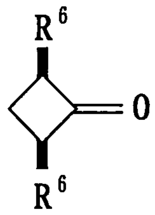

· · · · · (VI)

(wherein $R^6$ is independently a substituted or non-substituted alkyl group having a carbon number of 1 to 20 or a substituted or non-substituted aryl group having a carbon number of 6 to 15);

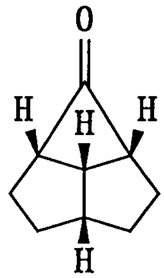

· · · · · (VII)

Concretely, the cyclic ketone compound includes 3-phenyl cyclobutanone, 3-(p-chlorophenyl) cyclobutanone, 3-(p-methoxyphenyl) cyclobutanone, 3-(2-naphthyl) cyclobutanone and 3-octyl cyclobutanone.

In the other preferable embodiment of the invention, the lactone compound is represented by the following formula (VIII), (IX) or (X):

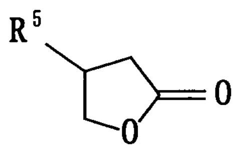

· · · · · (VIII)

(wherein $R^5$ is the same meaning as mentioned above);

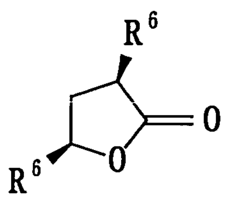

· · · · · (IX)

(wherein $R^6$ is the same meaning as mentioned above);

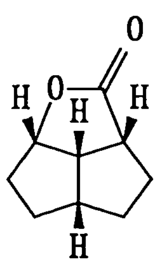

· · · · · (X)

Concretely, the lactone compound includes β-phenyl-γ-butyrolactone, β-(p-chlorophenyl)-γ-butyrolactone,

β-(p-methoxyphenyl)-γ-butyrolactone, β-(2-naphthyl)-γ-butyrolactone and (3-octyl-γ-butyrolactone.

In a further preferable embodiment of the invention, the Baeyer-Villiger oxidation is conducted in at least one organic solvent. Concretely, the organic solvent includes 1,2-dichloroethane, dichloromethane, 1,4-dioxane, diethyl ether, ethyl acetate, ethanol, acetone, dimethylformamide (DMF), 1,2-dimethoxyethane (DME) and tetrahydrofuran (THF).

According to the production method of the invention, both enantiomers of the optically active lactone compound can be also produced. The thus obtained optically active lactone compounds are useful as a chiral building block for the synthesis of medicines and agrochemicals.

## DETAILED DESCRIPTION OF THE INVENTION

The invention will be explained in detail below. The production method of the optically active lactone compound according to the invention comprises using a complex in which Pd or Pt is a central metal and a ligand is selected from the group consisting of a compound represented by the formula (I), (II), (III) or (IV), and its enantiomer as a catalyst, and conducting a Baeyer-Villiger oxidation of a cyclic ketone compound with at least one oxidizer selected from the group consisting of hydrogen peroxide, urea-hydrogen peroxide adduct (UHP) and alkyl hydroperoxide. In this method, the Baeyer-Villiger oxidation may be carried out by preparing the complex from a compound containing Pd or Pt and a ligand in an organic solvent, and further adding a cyclic ketone compound and an oxidizer to the organic solvent containing the complex without separating the complex from the organic solvent. In this case, it is possible to shorten the production process and reduce the production cost.

The Baeyer-Villiger oxidation used in the production method of the invention is also referred to as a Baeyer-Villiger reaction or a Baeyer-Villiger rearrangement, and is a reaction of producing an ester by an oxidation of a ketone with a peracid or a peroxide. The lactone compound is obtained by subjecting the cyclic ketone compound to this reaction. Further, the optically active lactone compound is obtained by enantioselectively subjecting the prochiral cyclic ketone to the above reaction.

The Baeyer-Villiger oxidation is a two-step reaction; (i) nucleophilic addition of a peroxide compound to a carbonyl group giving a Criegee adduct and (ii) the rearrangement of the Criegee adduct to an ester (or a lactone) by migration of C-C bond to proximal oxygen atom of the peroxide unit.

When the σ-orbital of the C-C bond interacts with the $\sigma^*$-orbital of the O-O bond, the rearrangement of the Criegee adduct to the lactone which is irreversible and rate-determining step proceeds smoothly. Therefore, it is expected that highly enantioselective Baeyer-Villiger oxidation is achieved if one of the two σ-bonds interacts with the $\sigma^*$-bond enantiotopos-selectively.

The Criegee adduct is a kind of $\eta^2$ ligand and makes a chelate with a metal ion possessing two vacant cis-coordination sites adjacent to each other. Thus, it is expected that the conformation of the Criegee adduct can be regulated to allow the enantiotopos-selective $\sigma$-$\sigma^*$ interaction if the Criegee adduct makes a chelate with a metal ion having an appropriate chiral ligand. In the production method of the invention, the conformation of the Criegee adduct making the chelate is regulated to allow the enantiotopos-selective $\sigma$-$\sigma^*$ interaction to give the optically active lactone compound owing to the use of the Pd complex or Pt complex having the specific ligand as mentioned later.

The complex used as a catalyst in the invention lies in that Pd or Pt is a central metal and a ligand is selected from the group consisting of a compound represented by the formula (I), (II), (III) or (IV), and its enantiomer. Moreover, both of optically active lactone enantiomers are obtained by properly using the compounds of the formulae (I) - (IV) and their enantiomers as a ligand.

In the formula (I), $R^1$ is a linear or branched alkyl group having a carbon number of 1 to 10 provided that a hydrogen atom of the alkyl group may be substituted with t-butyldimethylsiloxy (OTBS) group. As the linear or branched alkyl group having a carbon number of 1 to 10, mention may be made of i-propyl group, methyl group, cyclohexyl group, i-butyl group, n-pentyl group and so on. As $R^1$ in the formula (I), i-propyl group (i-pr) and 1-methyl-1-(t-butyldimethylsiloxy) ethyl group [$CMe_2$(OTBS)] are preferable, and i-propyl group is particularly preferable.

When the ligand of the complex is the compound represented by the formula (II), the complex is concretely represented by the following formula (II-M).

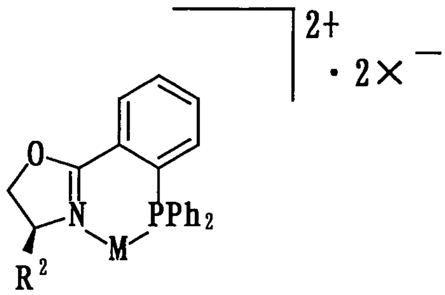

····· (II-M)

In the formulae (II) and (II-M), $R^2$ is an aryl group having a carbon number of 6 to 10 or a linear or branched alkyl group having a carbon number of 1 to 10. As the aryl group having a carbon number of 6 to 10, mention may be made of phenyl group, p-tolyl group, p-(trifluoromethyl) phenyl group, p-chlorophenyl group, 3,5-dimethylphenyl group and so on. As the linear or branched alkyl group having a carbon number of 1 to 10, mention may be made of t-butyl group, i-propyl group, methyl group, i-butyl group and so on. As $R^2$ in the formulae (II) and (II-M), phenyl group (Ph) and t-butyl group (t-Bu) are preferable, and t-butyl group is particularly preferable.

In the formula (III), $R^3$ is independently a linear or branched alkyl group having a carbon number of 1 to 10 provided that a hydrogen atom of the alkyl group may be substituted with t-butyldimethylsiloxy (OTBS) group. As the linear or branched alkyl group having a carbon number of 1 to 10, mention may be made of methyl group, i-propyl group, cyclohexyl group, i-butyl group, n-butyl group, n-pentyl group and so on. As $R^3$ in the formula (III), t-butyldimethylsiloxymethyl group and 1-methyl-1-(t-butyldimethylsiloxy) ethyl group are preferable.

When the ligand of the complex is the compound represented by the formula (IV), the complex is concretely represented by the following formula (IV-M).

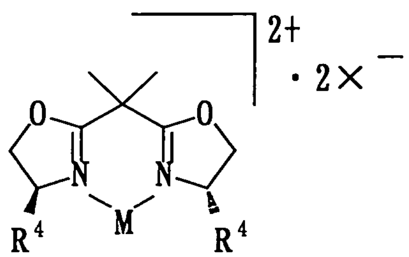

····· (IV-M)

In the formulae (IV) and (IV-M), $R^4$ is independently an aralkyl group having a carbon number of 7 to 11 or a linear or branched alkyl group having a carbon number of 1 to 10. As the aralkyl group having a carbon number of 7 to 11, mention may be made of benzyl group, 2,4,6-trimethylphenylmethyl group, 4-methylphenylmethyl group, 3,5-dimethylphenylmethyl group and so on.

As the linear or branched alkyl group having a carbon number of 1 to 10, mention may be made of t-butyl group, i-propyl group, methyl group, i-butyl group and so on. As $R^4$ in the formulae (IV) and (IV-M), benzyl group ($CH_2Ph$) and t-butyl group (t-Bu) are preferable.

In the formulae (II-M) and (IV-M), M is Pt or Pd, and $X^-$ is a monovalent anion (counter ion). As M, Pd is preferable. As $X^-$, $SbF_6^-$ and $BF_4^-$ are preferable, and $SbF_6^-$ is particularly preferable.

An amount of the catalyst used in the invention is a range of 1 to 10 mol%, preferably 4 to 6 mol% per the molar amount of the cyclic ketone as a substrate.

In the production method of the invention, the ligand regulates the face-selectivity in nucleophilic attack of the oxygen atom in the oxidizer bonded to the central metal of the complex to the carbon atom in the carbonyl group of the substrate and the enantiotopos-selectivity in the rearrangement by controlling the conformation of the Criegee adduct produced by the nucleophilic attack to give an optically active product.

The cyclic ketone compound used in the invention is a prochiral cyclic ketone compound producing an asymmetric carbon through the Baeyer-Villiger oxidation, which includes, for example, compounds represented by the formulae (V), (VI), (VII) and so on. The term "prochiral cyclic ketone compound" used herein means cyclic ketones producing an asymmetric carbon through the reaction.

As the alkyl group in $R^5$ of the formula (V), mention may be made of methyl, ethyl, propyl, isopropyl, butyl, isobutyl, t-butyl, pentyl, isopentyl, neopentyl, hexyl, heptyl, octyl, 2-ethylhexyl, nonyl, decyl, undecyl, dodecyl, tridecyl,

isotridecyl, myristyl, palmityl, stearyl, icocyl, docosyl and so on.

As the aryl group in $R^5$ of the formula (V), mention may be made of phenyl, toluyl, xylyl, cumenyl, mesityl, ethylphenyl, propylphenyl, butylphenyl, pentylphenyl, hexylphenyl, heptylphenyl, octylphenyl, nonylphenyl, 1-naphthyl, 2-naphthyl and so on.

Each of the above alkyl group and aryl group may be substituted with a halogen, an alkoxy group having a carbon number of 1 to 4, or the like.

The alkyl group and aryl group in $R^6$ of the formula (VI) are the same as mentioned in $R^5$ of the formula (V). Also, each of the alkyl group and aryl group may be substituted with a halogen, an alkoxy group having a carbon number of 1 to 4, or the like.

The oxidizer used in the invention is at least one of urea-hydrogen peroxide adduct (UHP), hydrogen peroxide and alkyl hydroperoxide. As the alkyl hydroperoxide, mention may be made of t-butyl hydroperoxide, cumyl hydroperoxide and so on. Among them, urea-hydrogen peroxide adduct (UHP) and hydrogen peroxide are preferable in the production method of the invention, and urea-hydrogen peroxide adduct (UHP) is particularly preferable. These oxidizers coordinate the central metal of the complex and attack the carbonyl group in the substrate to form a chelated Criegee adduct. An amount of the oxidizer used is within a range of 1 to 2 equivalent, preferably 1.2 to 1.3 equivalent per the cyclic ketone as a substrate.

The product according to the invention, optically active lactone compound is produced by the asymmetric Baeyer-Villiger oxidation of the prochiral cyclic ketone compound. The optically active lactone compound corresponds to the aforementioned cyclic ketone and includes, for example, compounds represented by the formulae (VIII), (IX), (X) and so on.

The optical purity used as a measure of the purity in the optically active isomer according to the invention is represented by the following equation.

$$\text{Optical purity (\%ee)} = \frac{|\alpha|_{D}}{|\alpha|_{Dmax}} = \frac{|R - S| \times 100}{R + S} \text{ or } \frac{|S - R| \times 100}{R + S} \text{ Enantiomeric excess (\%ee)}$$

wherein $[\alpha]_D$ is a specific rotation of a sample, $[\alpha]_{Dmax}$ is a specific rotation of an optically pure substance, R is a ratio of R-isomer occupied in the sample, and S is a ratio of S-isomer occupied in the sample. Therefore, the optical purity is coincident with the enantiomeric excess. When the ratios of R-isomer and S-isomer are the same, that is, when the sample is a racemate, the optical purity is 0 %ee. The optical purity (enantiomeric excess) of the reaction product can be measured by means of a high performance liquid chromatography (HPLC) using an optically active column.

The production method of the invention is usually carried out in an organic solvent. The organic solvent includes halogenated hydrocarbons such as 1,2-dichloroethane, dichloromethane ($CH_2Cl_2$) and the like; ethers such as 1,4-dioxane, diethyl ether ($Et_2O$), 1,2-dimethoxyethane (DME), tetrahydrofuran (THF) and the like; esters such as ethyl acetate (AcOEt) and the like; alcohols having a carbon number of 1 to 3 such as methanol, ethanol (EtOH), isopropanol and the like; ketones such as acetone and the like; and amides such as dimethylformamide (DMF) and the like. Among them, 1,2-dimethoxyethane (DME) and tetrahydrofuran (THF) are preferable in view of enhancing the enantiomeric excess of the product. An amount of the organic solvent used is a range of 1 to 10 mL, preferably 4 to 5 mL per 1 mmol of the cyclic ketone as a substrate.

The production method of the invention can be carried out at room temperature. When the reaction is carried out below room temperature, for example, at -20°C to -80°C, the enantiomeric excess is enhanced though the reaction rate is slow. Therefore, the latter temperature is preferable in view of enhancing the optical purity of the product.

In the invention, the optically active lactone compound can be produced by stirring a mixed solution of the cyclic ketone compound, the oxidizer, the organic solvent and the catalyst. The stirring method is not particularly limited so long as the uniformity of the mixed solution is ensured, so that any well-known methods can be applied. Also, the reaction time is not particularly limited and is properly selected in accordance with the reaction temperature. It is preferable that when the reaction temperature is high, the reaction time is short, while when the reaction temperature is low, the reaction time is long.

The following examples are given in illustration of the invention and are not intended as limitations thereof.

(Example 1)

To a solution of 1,5-cyclooctadiene platinum dichloride [(COD)$PtCl_2$] (2.2 mg, 5.0 μmol) in 1,2-dichloroethane (0.4 mL) is added a compound represented by the formula (I) in which $R^1$ is isopropyl group (2.3 mg, 5.5 μmol) under a nitrogen atmosphere and stirred at room temperature for 1 hour.
The resulting mixture is added to another flask previously containing silver tetrafluoroborate [$AgBF_4$] (1.9 mg, 10 μmol) under a nitrogen atmosphere, which is further stirred at room temperature for 1 hour, and filtered through a pad of

Celite to give a complex solution. The resulting complex is represented by the following formula (Ia-1).

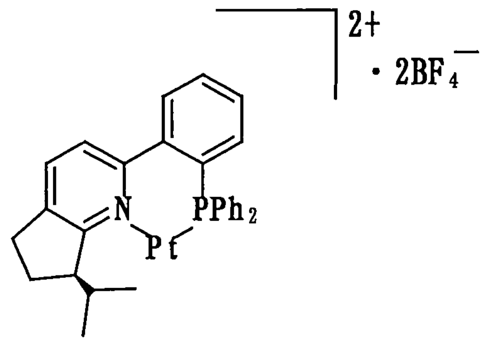

· · · · · (Ia-1)

To the resulting complex solution is added 3-phenylcyclobutanone (15.2 mg, 0.1 mmol). Then, to the resulting solution is added urea-hydrogen peroxide adduct (UHP) (12.2 mg, 0.13 mmol) and further stirred at room temperature for 24 hours. After the completion of the stirring, the resulting mixture is concentrated on a rotary evaporator and is chromatographed on a silica gel using a mixed solution of hexane/ethyl acetate (= 9/1) to obtain β-phenyl-γ-butyrolactone (13.3 mg, yield: 79%). As the enantiomeric excess of this product is determined by a high performance liquid chromatography (HPLC) using a DAICEL CHIRALPAK AD-H column and hexane/isopropanol(= 95/5), it is 7 %ee. Moreover, the absolute configuration of the product is determined by comparison of the elution time with the authentic sample (See Uchida T., Katsuki T., Ito K., Akashi S., Ishii A. and Kuroda T., Helv. Chim. Acta., 2002, 85, 3078). The results are shown in Table 1.

(Example 2)

The same procedure as in Example 1 is repeated except that an aqueous solution of 30% hydrogen peroxide (15 μL, content of hydrogen peroxide: 0.13 mmol) is used instead of the urea-hydrogen peroxide adduct (UHP) (12.2 mg, 0.13 mmol). The results are shown in Table 1.

(Example 3)

To a solution of bis(benzonitrile)palladium(II) chloride (1.9 mg, 5.0 μmol) in 1,2-dichloroethane (0.4 mL) is added a compound represented by the formula (I) in which $R^1$ is isopropyl group (2.3 mg, 5.5 μmol) under a nitrogen atmosphere and stirred at room temperature for 1 hour. The resulting mixture is added to another flask previously containing silver tetrafluoroborate $[AgBF_{4]}$ (1.9 mg, 10 μmol) under a nitrogen atmosphere, and further stirred at room temperature for 1 hour to synthesize a complex represented by the following formula (Ia-2-1) as a complex solution likewise Example 1.

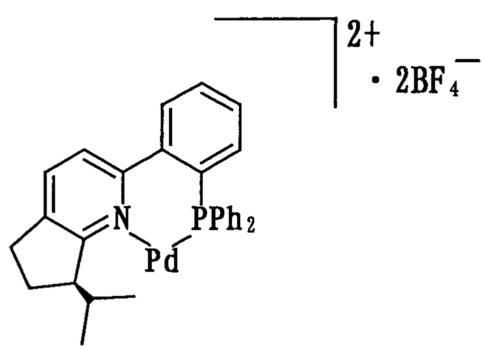

· · · · · (Ia-2-1)

To the resulting complex solution is added 3-phenylcyclobutanone (15.2 mg, 0.1 mmol). Then, the resulting solution is added with urea-hydrogen peroxide adduct (UHP) (12.2 mg, 0.13 mmol) and further stirred at room temperature for 20 hours. The resulting mixture is analyzed by the same manner as in Example 1. The results are shown in Table 1.

(Example 4)

The same procedure as in Example 3 is repeated except that silver hexafluoroantimonate [$AgSbF_6$] (3.4 mg, 10 μmol) is used instead of silver tetrafluoroborate [$AgBF_4$] (1.9 mg, 10 μmol) to synthesize a complex represented by the following formula (Ia-2-2). The reaction is also carried out under the same conditions as in Example 1 except that the reaction time is 2 hours and the product is analyzed by the same manner as in Example 1. The results are shown in Table 1.

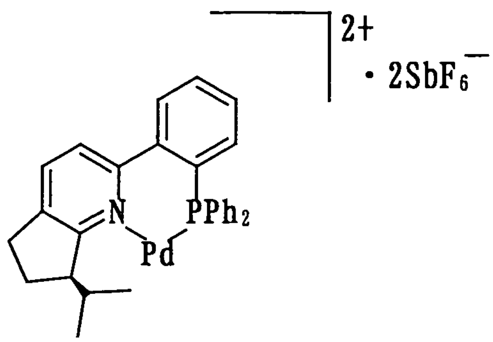

· · · · · (Ia-2-2)

(Example 5)

The same procedure as in Example 1 is repeated except that the complex of the formula (Ia-2-1) is used as a catalyst, and an aqueous solution of 30% hydrogen peroxide (15 μL, content of hydrogen peroxide: 0.13 mmol) is used as an oxidizer, and the reaction time is 20 hours. The results are shown in Table 1.

(Example 6)

The same procedure as in Example 3 is repeated except that a compound represented by the formula (I) in which $R^1$ is 1-methyl-1-(t-butyldimethylsiloxy) ethyl group (3.0 mg, 5.5 μmol) is used as a ligand instead of the compound represented by the formula (I) in which $R^1$ is isopropyl group (2.3 mg, 5.5 μmol) to synthesize a complex represented by the following formula (Ib). Also, the reaction is carried out under the same conditions as in Example 1 except that the complex of the formula (Ib) is used as a catalyst and the reaction time is 45 hours, and the product is analyzed by the same manner as in Example 1. The results are shown in Table 1.

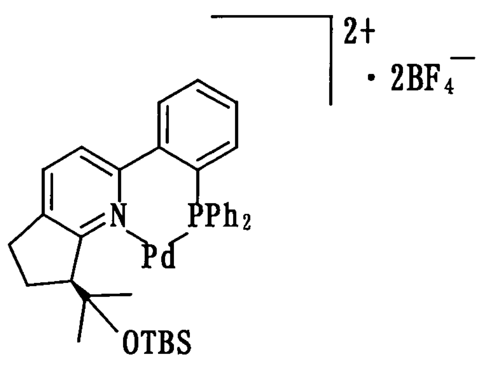

· · · · · (Ib)

(Example 7)

The same procedure as in Example 3 is repeated except that a compound represented by the formula (II) in which $R^2$ is phenyl group (1.9 mg, 5.5 μmol) is used as a ligand instead of the compound represented by the formula (I) in which $R^1$ is isopropyl group (2.3 mg, 5.5 μmol) to synthesize a complex represented by the following formula (IIa). Also, the reaction is carried out under the same conditions as in Example 1 except that the complex of the formula (IIa) is used as a catalyst and the reaction time is 17 hours, and the product is analyzed by the same manner as in Example 1. The results are shown in Table 1.

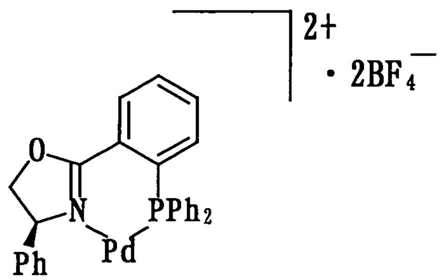

· · · · · (IIa)

(Example 8)

The same procedure as in Example 3 is repeated except that a compound represented by the formula (II) in which $R^2$ is t-butyl group (2.1 mg, 5.5 μmol) is used as a ligand instead of the compound represented by the formula (I) in which $R^1$ is isopropyl group (2.3 mg, 5.5. μmol) to synthesize a complex represented by the following formula (IIb). Also, the reaction is carried out under the same conditions as in Example 1 except that the complex of the formula (IIb) is used as a catalyst and the reaction time is 18 hours, and the product is analyzed by the same manner as in Example 1. The results are shown in Table 1.

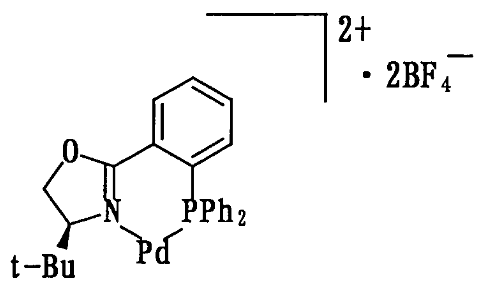

· · · · · (IIb)

Moreover, a synthetic example of a Pd complex having substantially the same ligand as the complexes of the formulae (IIa) and (IIb) in spite of having a different counter anion is described in J. Sprinz, M. Kiefer, G. Helmchen, M. Reggelin, G. Huttner, O. Walter and L. Zscinal, Tetrahedron Letters, 1994, 35, 1523-1526.

(Example 9)

The same procedure as in Example 3 is repeated except that a compound represented by the formula (III) in which every $R^3$ is 1-methyl-1-(t-butyldimethylsiloxy) ethyl group (3.2 mg, 5.5 μmol) is used as a ligand instead of the compound represented by the formula (I) in which $R^1$ is isopropyl group (2.3 mg, 5.5 μmol) to synthesize a complex represented by the following formula (IIIa). Also, the reaction is carried out under the same conditions as in Example 1 except that the complex of the formula (IIIa) is used as a catalyst and the reaction time is 89 hours, and the product is analyzed by the same manner as in Example 1. The results are shown in Table 1.

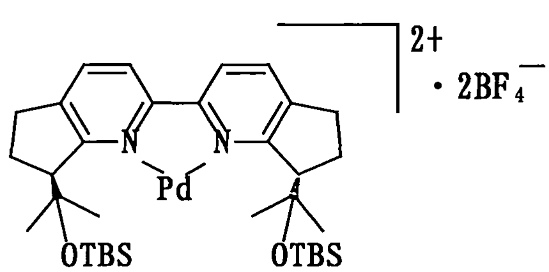

· · · · · (IIIa)

(Example 10)

The same procedure as in Example 3 is repeated except that a compound represented by the formula (IV)

in which every $R^4$ is benzyl group (2.0 mg, 5.5 μmol) is used as a ligand instead of the compound represented by the formula (I) in which $R^1$ is isopropyl group (2.3 mg, 5.5 μmol) to synthesize a complex represented by the following formula (IVa). Also, the reaction is carried out under the same conditions as in Example 1 except that the complex of the formula (IVa) is used as a catalyst and the reaction time is 12 hours, and the product is analyzed by the same manner as in Example 1. The results are shown in Table 1.

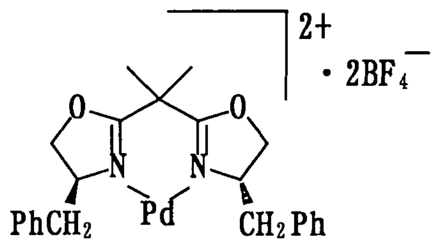

· · · · · (IVa)

Moreover, an example of preparing a Pd complex having the same ligand as the above complex in spite of having a different counter anion in a solution and using for another reaction is described in J. M. Zenner and R. C. Larock, J. Org. Chem., 1996, 64, 7312-7322.

(Example 11)

The same procedure as in Example 3 is repeated except that a compound represented by the formula (IV) in which every $R^4$ is t-butyl group (1.6 mg, 5.5 μmol) is used as a ligand instead of the compound represented by the formula (I) in which $R^1$ is isopropyl group (2.3 mg, 5.5 μmol) to synthesize a complex represented by the following formula (IVb). Also, the reaction is carried out under the same conditions as in Example 1 except that the complex of the formula (IVb) is used as a catalyst and the reaction time is 70 hours, and the product is analyzed by the same manner as in Example 1. The results are shown in Table 1.

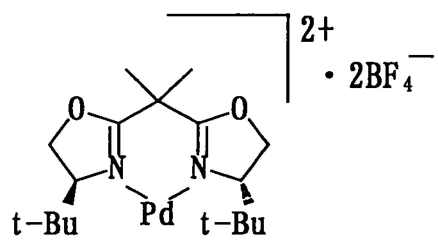

· · · · · (IVb)

Moreover, an example of using a Pd complex having the same ligand as the above complex in spite of having a different counter anion for another reaction is described in K. Ito, R. Kashiwagi, K. Iwasaki and T. Katsuki, Synlett, 1999, 1563-1566.

(Reference Example 1)

The same procedure as in Example 3 is repeated except that a compound represented by the following formula (XI) (3.4 mg, 5.5 μmol) is used as a ligand instead of the compound represented by the formula (I) in which $R^1$ is isopropyl group (2.3 mg, 5.5 μmol) to synthesize a complex represented by the following formula (XIa). Also, the reaction is carried out under the same conditions as in Example 1 except that the complex of the formula (XIa) is used as a catalyst and the reaction time is 3 hours, and the product is analyzed by the same manner as in Example 1. The results are shown in Table 1.

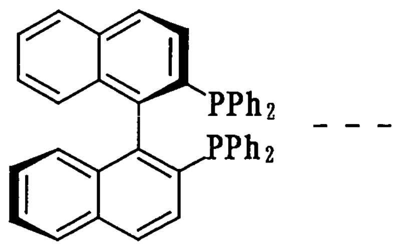

· · · · · (XI)

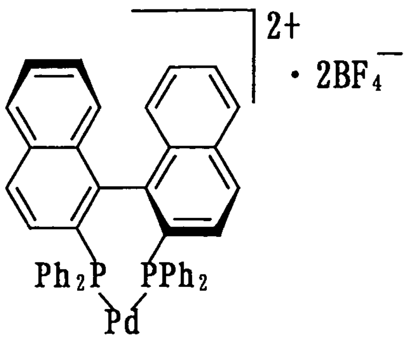

· · · · · (XIa)

(Reference Example 2)

The same procedure as in Reference Example 1 is repeated except that an aqueous solution of 30% hydrogen peroxide (15 μL, content of hydrogen peroxide: 0.13 mmol) is used instead of the urea-hydrogen peroxide adduct (UHP) (12.2 mg, 0.13 mmol). The results are shown in Table 1.

Table 1

| | Catalyst | Oxidizer | Reaction time (h) | Yield (%) | Enantiomeric excess (%ee) | Conformation |
|---|---|---|---|---|---|---|
| Example 1 | Formula (Ia-1) | UHP | 24 | 79 | 7 | R |
| Example 2 | Formula (Ia-1) | $H_2O_2$ | 24 | 84 | 3 | R |
| Example 3 | Formula (Ia-2-1) | UHP | 20 | 87 | 41 | R |
| Example 4 | Formula (Ia-2-2) | UHP | 2 | 100 | 47 | R |
| Example 5 | Formula (Ia-2-1) | $H_2O_2$ | 20 | 88 | 26 | R |
| Example 6 | Formula (Ib) | UHP | 45 | 52 | 8 | R |
| Example 7 | Formula (IIa) | UHP | 17 | 94 | 10 | R |
| Example 8 | Formula (IIb) | UHP | 18 | 100 | 38 | R |
| Example 9 | Formula (IIIa) | UHP | 89 | 35 | 9 | S |
| Example 10 | Formula (IVa) | UHP | 12 | 88 | 14 | S |

Table 1 (continued)

| | Catalyst | Oxidizer | Reaction time (h) | Yield (%) | Enantiomeric excess (%ee) | Conformation |
|---|---|---|---|---|---|---|
| Example 11 | Formula F(IVb) | UHP | 70 | 44 | 15 | R |
| Reference Example 1 | Formula (XIa) | UHP | 3 | 100 | 18 | R |
| Reference Example 2 | Formula (XIa) | $H_2O_2$ | 3 | 100 | 10 | R |

The reaction scheme corresponding to Examples 1 - 11 and Reference Examples 1 - 2 in Table 1 is shown as follows.

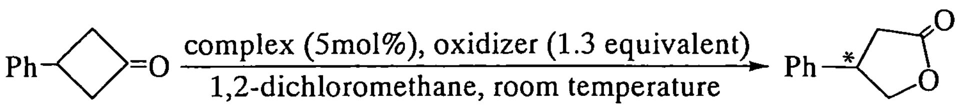


As seen from Table 1, the optically active lactone compound is obtained by using the above-mentioned complex as a catalyst and conducting the Baeyer-Villiger oxidation of the cyclic ketone with UHP or hydrogen peroxide as an oxidizer. As seen from the comparison of Example 1 with Example 3 and the comparison of Example 2 with Example 5, Pd is more preferable than Pt as a central metal. As seen from the comparison of Example 3 with Example 4, $SbF_6^-$ is more preferable than $BF_4^-$ as a counter ion. As seen from Examples 3 and 6 - 11, the compound represented by the formula (I) in which $R^1$ is isopropyl group and the compound represented by the formula (II) in which $R^2$ is t-butyl group are preferable as a ligand, and the compound represented by the formula (I) in which $R^1$ is isopropyl group is particularly preferable. As seen from the comparison of Example 1 with Example 2 and the comparison of Example 3 with Example 5, UHP is more preferable than $H_2O_2$ as an oxidizer in view of the enantiomeric excess.

(Examples 12 - 25)

An oxidization of 3-phenyl cyclobutanone is carried out by the same manner as in Example 1 except that the complex represented by the formula (Ia-2-2) is used as a catalyst with a solvent, a reaction temperature and a reaction time shown in Table 2. The results are shown in Table 2.

Table 2

| | Solvent | Reaction temperature (°C) | Reaction time (h) | Yield (%) | Enantiomeric excess (%ee) |
|---|---|---|---|---|---|
| Example 12 | $CH_2Cl_2$ | r.t. | 10 | 100 | 50 |
| Example 13 | 1,4-Dioxane | r.t. | 19 | 95 | 47 |
| Example 14 | $Et_20$ | r.t. | 18 | 94 | 44 |
| Example 15 | AcOEt | r.t. | 1.5 | 100 | 53 |
| Example 16 | EtOH | r.t. | 0.5 | 100 | 51 |
| Example 17 | Acetone | r.t. | 19 | 100 | 51 |
| Example 18 | DMF | r.t. | 23 | 98 | 48 |
| Example 19 | DME | r.t. | 0.5 | 100 | 57 |
| Example 20 | DME | -40 | 46 | 93 | 73 |
| Example 21 | THF | r.t. | 23 | 92 | 59 |
| Example 22 | THF | -20 | 18 | 100 | 73 |

Table 2 (continued)

| | Solvent | Reaction temperature (°C) | Reaction time (h) | Yield (%) | Enantiomeric excess (%ee) |
|---|---|---|---|---|---|
| Example 23 | THF | -40 | 24 | 100 | 78 |
| Example 24 | THF | -60 | 214 | 91 | 80 |
| Example 25 | THF | -80 | 186 | 98 | 60 |
| r.t. = room temperature | | | | | |

The reaction scheme corresponding to Examples 12 - 25 in Table 2 is shown as follows.

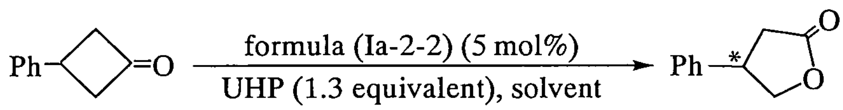


As seen from Table 2, DME and THF are preferable as a solvent in view of the enantiomeric excess. Also, it is seen that the optical purity is enhanced by reacting below room temperature, while the optical purity is lowered by reacting at excessive low temperature.

(Example 26)

The same procedure as in Example 24 is repeated except that 3-(p-chlorophenyl) cyclobutanone (0.1 mmol) is used instead of 3-phenyl cyclobutanone (0.1 mmol) and the reaction time is 208 hours. As a result, β-(p-chlorophenyl)-γ-butyrolactone is obtained as a product instead of β-phenyl-γ-butyrolactone. As the absolute configuration of the product is determined by the comparison of the elution time with the authentic sample, the product is mainly composed of R-isomer (See Uchida T., Katsuki T., Ito K., Akashi S., Ishii A. and Kuroda T., Helv. Chim. Acta., 2002, 85, 3078). The results are shown in Table 3. The reaction scheme is shown as follows.

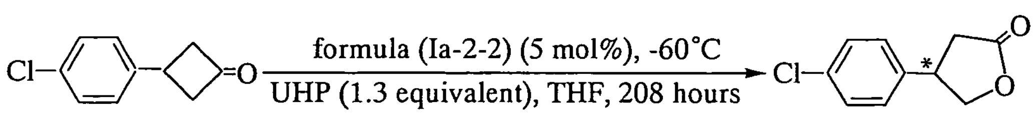


(Example 27)

The same procedure as in Example 24 is repeated except that 3-(p-methoxyphenyl) cyclobutanone (0.1 mmol) is used instead of 3-phenyl cyclobutanone (0.1 mmol) and the reaction time is 208 hours. As a result, β-(p-methoxyphenyl)-γ-butyrolactone is obtained as a product instead of β-phenyl-γ-butyrolactone. The results are shown in Table 3. The reaction scheme is shown as follows.

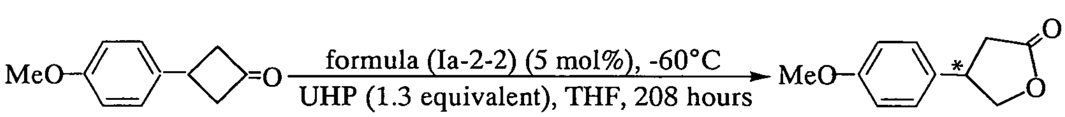


(Example 28)

The same procedure as in Example 24 is repeated except that 3-(2-naphthyl) cyclobutanone (0.1 mmol) is used instead of 3-phenyl cyclobutanone (0.1 mmol) and the reaction time is 211 hours. As a result, β-(2-naphthyl)-γ-butyrolactone is obtained as a product instead of β-phenyl-γ-butyrolactone. The results are shown in Table 3. The reaction scheme is shown as follows.

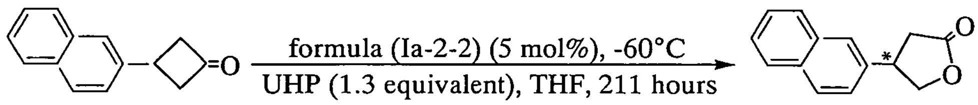


(Example 29)

The same procedure as in Example 24 is repeated except that 3-octyl cyclobutanone (0.1 mmol) is used instead of 3-phenyl cyclobutanone (0.1 mmol) and the reaction time is 211 hours. As a result, β-octyl-γ-butyrolactone is obtained as a product instead of β-phenyl-γ-butyrolactone. The enantiomeric excess of the product is analyzed by the high performance liquid chromatograph (HPLC) using a DAICEL CHIRALCEL OD-H column and an eluent of hexane/isopropanol (= 90/10) after the product is converted into the corresponding γ-hydroxy benzylamide according to the procedure described in Uchida T., Katsuki T., Ito K., Akashi S., Ishii A. and Kuroda T., Helv. Chim. Acta., 2002, 85, 3078. The results are shown in Table 3. The reaction scheme is shown as follows.

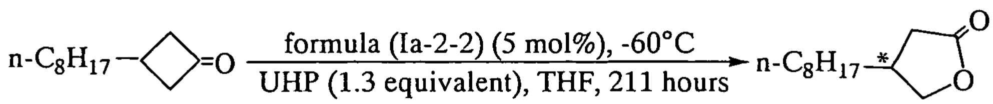


(Example 30)

The same procedure as in Example 23 is repeated except that the compound represented by the formula (VII) (0.1 mmol) is used instead of 3-phenyl cyclobutanone (0.1 mmol) and the reaction time is 44 hours. As a result, the compound represented by the formula (X) is obtained as a product instead of β-phenyl-γ-butyrolactone. The majority part of the product is (1S, 4R, 7R, 10S)-isomer. The results are shown in Table 3. The reaction scheme is shown as follows.

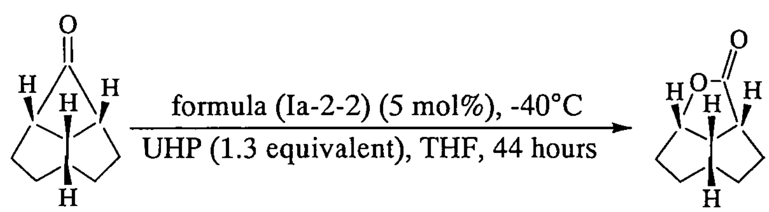


Table 3

| | Substrate | Reaction temperature (°C) | Reaction time (h) | Yield (%) | Enantiomeric excess (%ee) |
|---|---|---|---|---|---|
| Example 26 | Formula (V) $R^5$=p-$ClC_6H_4$ | -60 | 208 | 76 | 73 |
| Example 27 | Formula (V) $R^5$=p-$MeO_6H_4$ | -60 | 208 | 52 | 73 |
| Example 28 | Formula (V) $R^5$=p-$C_{10}H_7$ | -60 | 211 | 94 | 83 |
| Example 29 | Formula (V) $R^5$=n-$C_8H_{17}$ | -60 | 211 | 65 | 60 |
| Example 30 | Formula (VII) | -40 | 44 | 89 | >99 |

As seen form Table 3, the production method according to the invention can be applied to various cyclic ketones. Also, it is seen that the aryl group is preferable as $R^5$ in formula (V) in view of the enantiomeric excess. It is further seen that the lactone with a very high optical purity is obtained by applying the production method of the invention to a tricyclic ketone.

The optically active lactone compounds obtained according to the production method of the invention are useful as a chiral building block for the synthesis of medicines and agrochemicals.

## Claims

1. A method of producing an optically active lactone compound, comprises using as a catalyst a complex in which Pd or Pt is a central metal and a ligand is selected from the group consisting of a compound represented by the following formula (I), (II), (III) or (IV) and its enantiomer, and subjecting a cyclic ketone compound to a Baeyer-Villiger oxidation with at least one oxidizer selected from the group consisting of hydrogen peroxide, urea-hydrogen peroxide adduct and alkyl hydroperoxide:

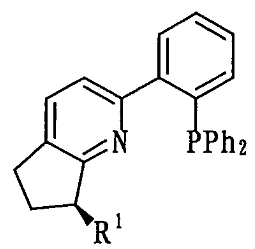

······ (I)

(wherein $R^1$ is a linear or branched alkyl group having a carbon number of 1 to 10 provided that a hydrogen atom of the alkyl group may be substituted with t-butyldimethylsiloxy group);

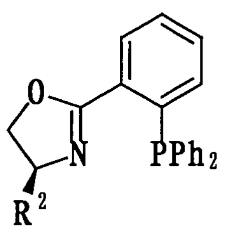

······ (II)

(wherein $R^2$ is an aryl group having a carbon number of 6 to 10 or a linear or branched alkyl group having a carbon number of 1 to 10);

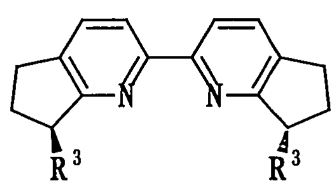

······ (III)

(wherein $R^3$ is independently a linear or branched alkyl group having a carbon number of 1 to 10 provided that a hydrogen atom of the alkyl group may be substituted with t-butyldimethylsiloxy group);

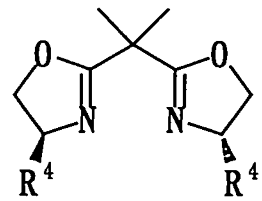

······ (IV)

(wherein $R^4$ is independently an aralkyl group having a carbon number of 7 to 11 or a linear or branched alkyl

group having a carbon number of 1 to 10).

**2.** A method according to claim 1, wherein a counter ion of the complex is $SbF_6^-$ or $BF_4^-$.

**3.** A method according to claim 1, wherein the central metal of the complex is Pd.

**4.** A method according to claim 1, wherein the ligand of the complex is a compound represented by the formula (I) in which $R^1$ is i-propyl group or 1-methyl-1-(t-butyldimethylsiloxy) ethyl group, or its enantiomer.

**5.** A method according to claim 1, wherein the ligand of the complex is a compound represented by the formula (II) in which $R^2$ is phenyl group or t-butyl group, or its enantiomer.

**6.** A method according to claim 1, wherein the ligand of the complex is a compound represented by the formula (III) in which $R^3$ is t-butyldimethylsiloxymethyl group or 1-methyl-1-(t-butyldimethylsiloxy)ethyl group, or its enantiomer.

**7.** A method according to claim 1, wherein the ligand of the complex is a compound represented by the formula (IV) in which $R^4$ is benzyl group or t-butyl group, or its enantiomer.

**8.** A method according to claim 1, wherein the complex comprises Pd as a central metal, a compound represented by the formula (I) in which $R^1$ is i-propyl group, or its enantiomer as a ligand and $SbF_6^-$ as a counter ion.

**9.** A method according to claim 1, wherein the cyclic ketone compound is represented by the following formula (V), (VI) or (VII):

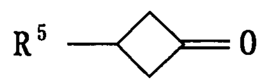

· · · · · (V)

(wherein $R^5$ is a substituted or non-substituted alkyl group having a carbon number of 1 to 20 or a substituted or non-substituted aryl group having a carbon number of 6 to 15);

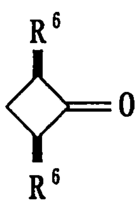

· · · · · (VI)

(wherein $R^6$ is independently a substituted or non-substituted alkyl group having a carbon number of 1 to 20 or a substituted or non-substituted aryl group having a carbon number of 6 to 15);

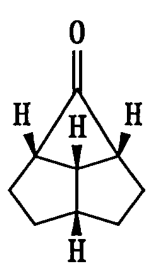

· · · · · (VII)

**10.** A method according to claim 9, wherein the cyclic ketone compound is 3-phenyl cyclobutanone, 3-(p-chlorophenyl) cyclobutanone, 3-(p-methoxyphenyl) cyclobutanone, 3-(2-naphthyl) cyclobutanone or 3-octyl cyclobutanone.

**11.** A method according to claim 1, wherein the lactone compound is represented by the following formula (VIII), (IX) or (X):

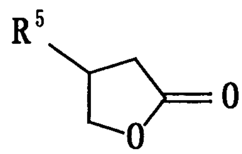

· · · · · (VIII)

(wherein $R^5$ is the same meaning as mentioned above);

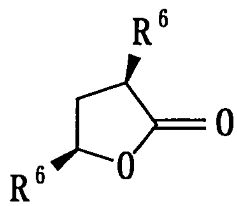

· · · · · (IX)

(wherein $R^6$ is the same meaning as mentioned above);

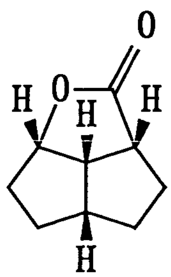

· · · · · (X)

**12.** A method according to claim 11, wherein the lactone compound is β-phenyl-γ-butyrolactone, β-(p-chlorophenyl)-γ-butyrolactone, β-(p-methoxyphenyl)-γ-butyrolactone, β-(2-naphthyl)-γ-butyrolactone or β-octyl-γ-butyrolactone.

**13.** A method according to claim 1, wherein the Baeyer-Villiger oxidation is conducted in at least one organic solvent.

**14.** A method according to claim 13, wherein the organic solvent is 1,2-dichloroethane, dichloromethane, 1,4-dioxane, diethyl ether, ethyl acetate, ethanol, acetone, dimethylformamide, 1,2-dimethoxyethane or tetrahydrofuran.

European Patent Office

# EUROPEAN SEARCH REPORT

Application Number
EP 04 25 0834

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | AKIRA WATANABE ET AL.: "HIGHLY ENANTIOSELECTIVE BAEYER-VILLIGER OXIDATION USING ZR(SALEN) COMPLEX AS CATALYST" TETRAHEDRON LETTERS., vol. 43, no. 25, 2002, pages 4481-5, XP004361180 ELSEVIER SCIENCE PUBLISHERS, AMSTERDAM., NL ISSN: 0040-4039 * page 4481 - page 4484 * --- | 1,9-14 | C07D315/00 |
| P,X | KATSUJI ITO ET AL.: "ASYMETRIC BAEYER-VILLIGER OXIDATION OF PROCHIRAL CYCLOBUTANONES" SYNLETT, vol. 5, 10 April 2003 (2003-04-10), pages 643-6, XP001189443 STUTTGART * page 643 - page 645 * ----- | 1-14 | |
| | | | TECHNICAL FIELDS SEARCHED (Int.Cl.7) |
| | | | C07D |

The present search report has been drawn up for all claims

| Place of search | Date of completion of the search | Examiner |
|---|---|---|
| THE HAGUE | 6 May 2004 | Francois, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)